# EUROPEAN PATENT APPLICATION

(11) **EP 2 174 681 A1**
(43) Date of publication of application: **14.04.2010**
(21) Application number: 08790714.3
(22) Date of filing: 27.06.2008
(51) Int. Cl.: A61M 5/315

(54) **PLUNGER ASSEMBLY FOR INJECTOR**

(30) Priority: 13.07.2007 JP 2007184327
(71) Applicant: Daikyo Seiko, LTD., Tokyo 131-0031 (JP)
(72) Inventor: KAWAMURA, Hideaki, Tokyo 131-0031 (JP); TOGASHI, Hiroshi, Tokyo 131-0031 (JP)
(74) Representative: Piésold, Alexander James
(86) International application number: PCT/JP2008/061749
(87) International publication number: WO 2009/011214

(57) **Abstract**

It is an object to provide a plunger assembly for a syringe
in which a piston can be held at the leading end of a plunger rod without fault and the piston together with the plunger rod can be pulled out in an integrated manner even in the case in which a pulling out operation is carried out in order to fill with a medicinal solution in a syringe barrel by pulling out the piston with the plunger rod.

## Description

### TECHNICAL FIELD

The present invention relates to a plunger assembly for a syringe for pushing out a medicinal solution retained in a syringe barrel.

### BACKGROUND ART

In general, a plunger assembly for a syringe is composed of a piston which is slidably fitted into a syringe barrel and a plunger rod which is screwed into an axis apart of the piston. The piston and the plunger rod are generally assembled in a fitting manner of a male screw and a female screw.

A piston which slides in a syringe barrel is formed by a rubber as a main component. To reduce a sliding resistance of the piston, silicone oil has been applied to the surface of the piston that is provided in some cases. However, in consideration of an influence of the silicone oil to a human body, the present patent applicant and so on has provided a laminate piston in which a tetrafluoroethylene resin film, an ethylene tetrafluoroethylene resin film, or a high molecular weight polyethylene resin film is laminated on the surface of a rubber piston.

Moreover, an actual field site of a medical treatment involves an operation of not only pushing out a piston but also pulling out a piston, more specifically an operation of confirming an introduction of a needle and filling with a medicinal solution in a syringe barrel by making a piston together with a plunger rod move in reverse to the base end side of a syringe barrel.

However, in the case in which a sliding resistance of the piston and a syringe barrel is large for a conventional plunger assembly for a syringe when a piston together with a plunger rod is pulled out, a piston is dropped out of a plunger rod in some cases.

In recent years, a syringe has a small capacity in order to lessen a burden of the patient, and a diameter of a syringe barrel has a tendency to be decreased. As a resultant event, a diameter of a piston is required to be extremely decreased. In the case in which a particularly thin piston is used, although a depression (a hole for a fitting) can be formed in a piston, it is difficult to form a minute female screw that can be fit to the inner wall of the piston in effect.
Patent document 1: Japanese Patent Application Laid-Open Publication No. 2000-140103

### DISCLOSURE OF THE INVENTION

### PROBLEMS TO BE SOLVED BY THE INVENTION

The present invention was made in consideration of such conditions, and an object of the present invention is to provide a plunger assembly for a syringe in which a force to pushing a piston is hardly applied in the case in which a piston and a plunger rod are engaged with each other, a piston is engaged with a plunger rod in a slidable manner after an engagement, and a piston can be held at the leading end of a plunger rod without fault and the piston together with the plunger rod can be pulled out in an integrated manner even in the case in which a pulling out operation is carried out in order to fill with a medicinal solution in a syringe barrel by making the piston together with the plunger rod move in reverse to the base end side of the syringe barrel for an extremely thin syringe having a small capacity in particular.

### MEANS FOR SOLVING THE PROBLEMS

A plunger assembly for a syringe in accordance with the present invention is characterized by comprising:
a piston that is slidably inserted into a syringe barrel; and
a plunger rod in which one end part thereof is inserted into an opened depression formed on the base end side of the piston to support the piston in an integrated manner,
wherein the depression of the piston is a hole in a generally cylindrical shape formed and opened in one direction, a male screw that comes into contact with an inner wall of the cylindrical hole of the piston in a sliding manner or that is screwed into the hole is formed at one end part of the plunger rod, and the piston is supported to the plunger rod in an integrated manner by inserting the male screw part into the depression.

The plunger assembly for a syringe in accordance with the present invention is **characterized in that** the piston is a laminate piston in which a resin film is laminated on the surface thereof.

The surface of the piston in the present specification includes not only a peripheral surface of the piston, that is, a surface that comes into contact with a chemical solution, and a sliding surface of the piston, but also a surface of the depression. Moreover, the above laminate piston represents pistons related to three modes of a piston in which only a peripheral surface is laminated, a piston in which only a surface of the depression is laminated, and a piston in which both the peripheral surface and the depression are laminated.

### EFFECT OF THE INVENTION

For the plunger assembly for a syringe in accordance with the present invention, even in the case in which a female screw is not formed on the piston, the plunger rod is fitted to the piston in a state that the male screw formed at an end part of the plunger rod comes into contact with an inner wall of the depression of the piston in a sliding manner or that the male screw is screwed into the depression, thereby preventing the piston from being dropped out of the plunger rod.

Moreover, it is unnecessary to form a female screw in the depression of the piston, thereby implementing the easy working and processing.

### BRIEF DESCRIPTION OF THE DRAWINGS

Fig. 1 is a cross-sectional view showing a fitting state of a syringe barrel that configures a syringe in accordance with an embodiment of the present invention and a plunger assembly for a syringe in accordance with the present invention.
Fig. 2 is a half cross-sectional view showing a laminate piston of the plunger assembly for a syringe shown in Fig. 1.
Fig. 3 is an elevation view showing a plunger rod of the plunger assembly for a syringe shown in Fig. 1.
Fig. 4 is an elevation view showing a plunger assembly for a syringe in the case in which a plunger rod is attached to the laminate piston shown in Fig. 2.
Fig. 5 is an elevation view showing another example of a plunger rod shown in Fig. 3.

### EXPLANATIONS OF LETTERS OR NUMERALS

- 2:: Syringe barrel
- 4:: Piston
- 6:: Plunger rod
- 6a:: Rod body
- 8:: Depression
- 10:: Plunger assembly for a syringe
- 12:: Male screw
- 12a:: Thread crest
- 12b:: Thread gullet
- 13:: Ring body
- 14:: Pressing flange

### BEST MODE OF CARRYING OUT THE INVENTION

A preferred embodiment (example) of the present invention will be described below in more detail.

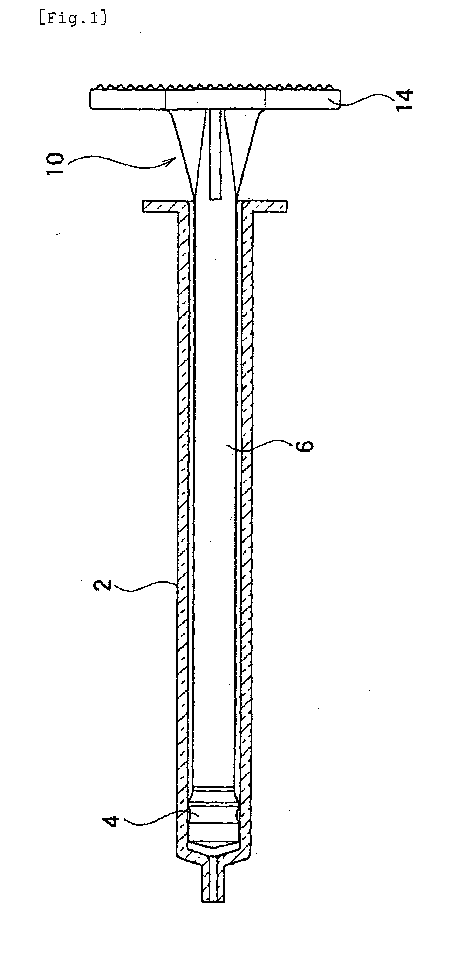

Fig. 1 is a cross-sectional view showing a plunger assembly for a syringe in accordance with an embodiment of the present invention.

A plunger assembly 10 for a syringe in accordance with an embodiment of the present invention is composed of a piston 4 that is inserted into a syringe barrel 2 in a slidable manner and a plunger rod 6 that is attached into the depression of the piston 4.

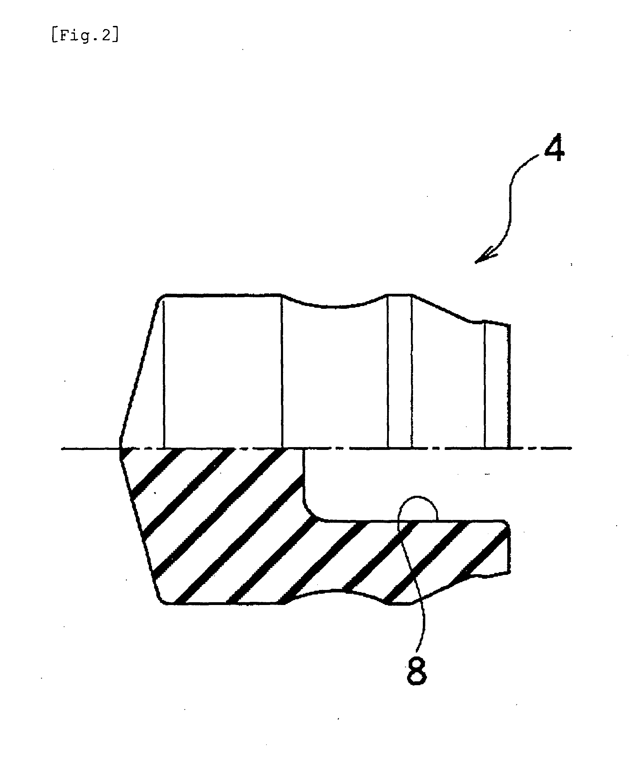

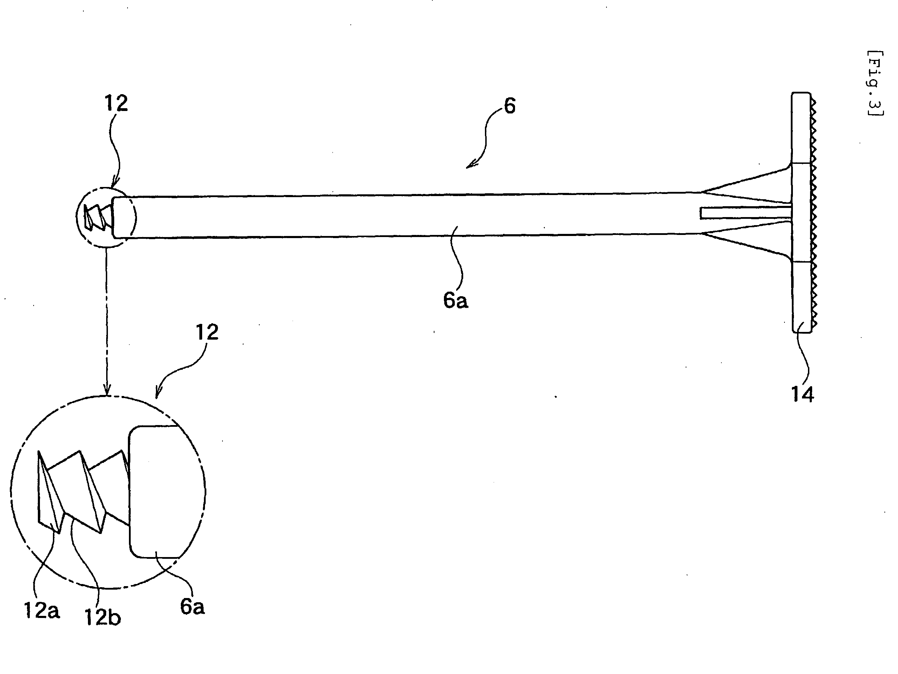

For the piston as shown in Fig. 2, a resin film is laminated to a peripheral surface of a rubber piston, and a depression 8 is formed in an axial direction on the base end side. It is preferable that an outer diameter of the piston 4 is in the range of 3.0 to 5.5 mm, an overall length is in the range of 4.0 to 7.0 mm, and an inner diameter of the depression 8 is in the range of 1.0 to 4.0 mm. As shown in Fig. 3, for the plunger rod 6, a male screw 12 that is inserted into the depression 8 of the piston 4 is formed at the leading end of a rod body 6a. Moreover, a pressing flange 14 is formed on the rear end side the plunger rod 6.

As shown by an enlarged view in Fig. 3, the male screw 12 is composed of a thread crest 12a and a thread gullet 12b in a spiral shape, and a diameter of the thread crest 12a is larger than an inner diameter of the depression 8 of the piston 4. Consequently, by pressing the male screw 12 into the depression 8 of the piston 4, the male screw 12 of the plunger rod 6 is cut into the depression 8 of the piston 4 that is relatively soft.

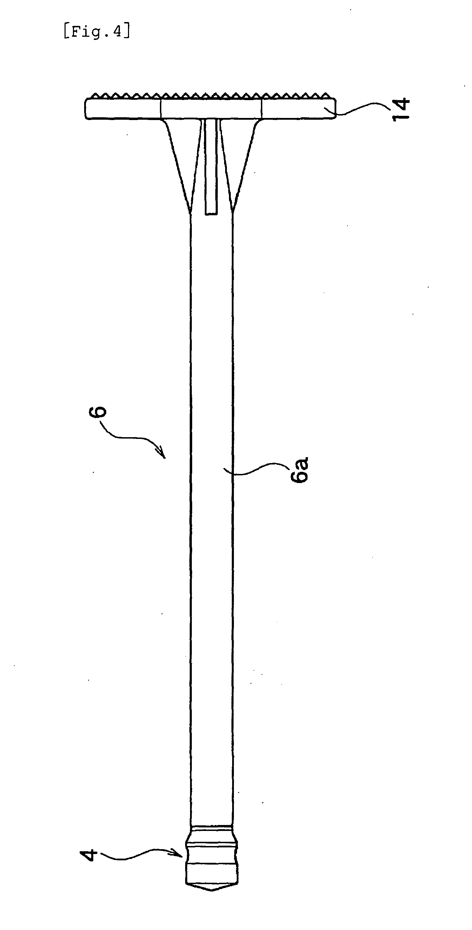

By the above configuration, the piston 4 is engaged with the plunger rod 6 as shown in Fig. 4.

Consequently, in the case in which the piston 4 is engaged with the plunger rod 6 in accordance with the present invention, the male screw 12 of the plunger rod 6 shown in Fig. 3 is disposed opposite to the base end side of the piston 4 shown in Fig. 2 at first, and the male screw 12 of the plunger rod 6 is screwed into the depression 8 of the piston 4 while a slight force is applied. At this time, in the depression 8 of the piston 4, the thread crest 12a extending in a screw pattern for the male screw 12 is fitted to the inner wall of the depression 8 and is cut into the inner wall, whereby the piston 4 can be engaged with the plunger rod 6 without fault.

The relationship between an inner diameter of the depression 8 of the piston 4 and a diameter of the thread crest 12a depends on an inner diameter of the syringe barrel 2 and a shape and a material of the piston 4. It is preferable that a diameter of the thread crest 12a of the male screw 12 is larger than an inner diameter of the depression 8 of the piston 4 by the range of 10 to 25%. In the case in which a degree is 10% or less, the piston 4 is easily dropped out of the plunger rod 6 undesirably. On the other hand, in the case in which a degree is 25% or larger, a sliding resistance of the piston 4 is increased undesirably.

For a length of the depression 8 of the piston 4, as the depression 8 is longer, an engaging property of the piston 4 with the plunger rod 6 is further improved. However, a resistance force that is applied to the male screw 12 on screwing is increased. Consequently, in the case in which a length of the depression 8 is a certain value or larger, the male screw 12 is broken disadvantageously before the screwing is completed. To solve the problem, it is preferable that a film is laminated or coated on the depression 8 as described later. By this configuration, a resistance force that is applied to the male screw 12 on screwing can be reduced due to a sliding property of a film, thereby improving a screwing property.

In the case in which a film is laminated or coated on the depression 8, the piston 4 is easily dropped out of the plunger rod 6 on pulling out. However, since a surface of the depression 8 is hardened due to a lamination of a film, the piston 4 is hard to be dropped out. Consequently, in the case in which a film is laminated on the depression 8 and a length of the depression 8 is larger, the piston 4 is hard to be dropped out in comparison with the case in which a film is not laminated on the depression 8.

Moreover, it is preferable that a length of the depression 8 is 70% or less of a length of the piston 4. In the case in which a length of the depression 8 is more than 70% of a length of the piston 4, a sealing performance of the piston 4 is deteriorated, and a molding performance of the male screw 12 of the plunger rod 6 is deteriorated disadvantageously.

By the plunger assembly 10 for a syringe in accordance with an embodiment of the present invention, it is unnecessary that a female screw is formed in the depression 8 of the piston 4. Consequently, the present invention can be applied to an extra thin piston 4. As an example, the present invention can be applied to an extra thin syringe piston that has a small capacity of 5 ml or less and that is hard to have a substantive female screw inside the piston, for instance a syringe piston that has a small capacity of 0.5 ml or less and that has a syringe barrel inner diameter of 5 mm or less.

The piston 4 can be engaged with the plunger rod 6 without fault by the male screw 12 of the plunger rod 6. Consequently, in the case in which an operation of pulling out the piston 4 is carried out, the piston 4 can be prevented from being dropped out of the plunger rod 6.

As a material of the syringe barrel 2 in accordance with the present invention, a plastic is used in general, and there can be mentioned for instance a cyclic olefin series resin, a cyclic olefin ethylene copolymer, a polyethylene terephthalate series resin, a polystyrene resin, and a polyethylene terephthalate resin. In particular, it is preferable to use a cyclic olefin series resin and a cyclic olefin ethylene copolymer that have a high transparency and an excellent heat resistance property and that have no chemical interaction with a pharmacological product (for instance, those used for a hygiene container disclosed in Patent Publication No. 2914826).

As a material of the piston 4 before a resin film is laminated on the piston 4, any one of a rubber material and an elastomer material, which have been used for a production of a piston, can be used, and the material is not restricted in particular. As a material of the piston 4, there can be mentioned for instance a butyl series rubber such as a butyl rubber, a chlorinated butyl rubber, a brominated butyl rubber, and a divinylbenzene copolymerization butyl rubber; a conjugated diene series rubber such as a polyisoprene rubber, (high to low cis 1, 4 bonding), a polyisobutylene rubber, a polybutadiene rubber (high to low cis 1, 4 bonding), and a styrene-butadiene copolymerization rubber; and an ethylene-propylene-diene terpolymerization rubber (EPDM).
As an elastomer, an olefin series copolymer rubber (elastomer) and/or olefin series plastics can be mentioned for instance.

The piston 4 in accordance with the present invention is produced by using an elastomer or a crosslinking rubber composition (compound) that is obtained by kneading the above rubber material and a compounding agent such as a crosslinking agent, a filler and/or a reinforcing agent, a coloring agent, and an age resister as needed and by a publicly known molding method such as a compression molding and an injection molding for a piston. A compounding agent to be used is not restricted in particular, and can be a compounding agent that has been used in producing a rubber stopper or a piston for a pharmacological product or medical treatment tools.

The piston 4 for a syringe in accordance with the present invention is a laminate piston, a surface that comes into contact with a chemical solution or a sliding surface of the piston is laminated by a plastic film made of a material such as a fluorine resin, ultrahigh molecular weight polyethylene, polyethylene, polypropylene, polyester, and nylon. In particular, from a point of view of stability, a water repellency, and a sliding property of a piston part that comes into contact with a solution, it is preferable that the periphery of the piston is laminated or coated by a fluorine resin film.

In the case in which the piston 4 is coated with silicone oil, the silicone oil comes in the depression 8, whereby the plunger rod 6 is dropped out disadvantageously in some cases. As a fluorine resin in accordance with the present invention, there can be selected for instance PTFE (polytetrafluoroethylene), ETFE (ethylene-tetrafluoroethylene copolymer), PFE (perfluoro alkoxyalkane), PFA (perfluoro ethylene propene copolymer), PVDF (polyvinylidene fluoride), and an alloy of those and other polymer as needed. From a point of view of a sliding property and a low reactivity, PTFE (polytetrafluoroethylene) is preferable in particular.

It is preferable that the depression 8 of the piston 4 for a syringe in accordance with the present invention is laminated or coated with a film as a point of view of a screwing property and an engaging property. As a material of a film that is laminated or coated on the depression 8, there can be used for instance a fluorine resin, ultrahigh molecular weight polyethylene, polyethylene, polypropylene, polyester, and nylon similarly to other parts of the piston 4 as described above. From a point of view of a suitable sliding property, a low reactivity, and a cost, ultrahigh molecular weight polyethylene is preferable in particular. A material of a film that is laminated or coated on the depression 8 and a material of a film that is laminated or coated on other parts of the piston 4 can be the same or can be different from each other.

A syringe in which a plunger assembly for a syringe in accordance with the present invention is used includes a syringe having two rooms as disclosed in Japanese Patent Application Laid-Open Publication No. 9-308688 in addition to a normal syringe having one room.

While the preferred embodiments in accordance with the present invention have been described above, the present invention is not restricted to the above embodiments.

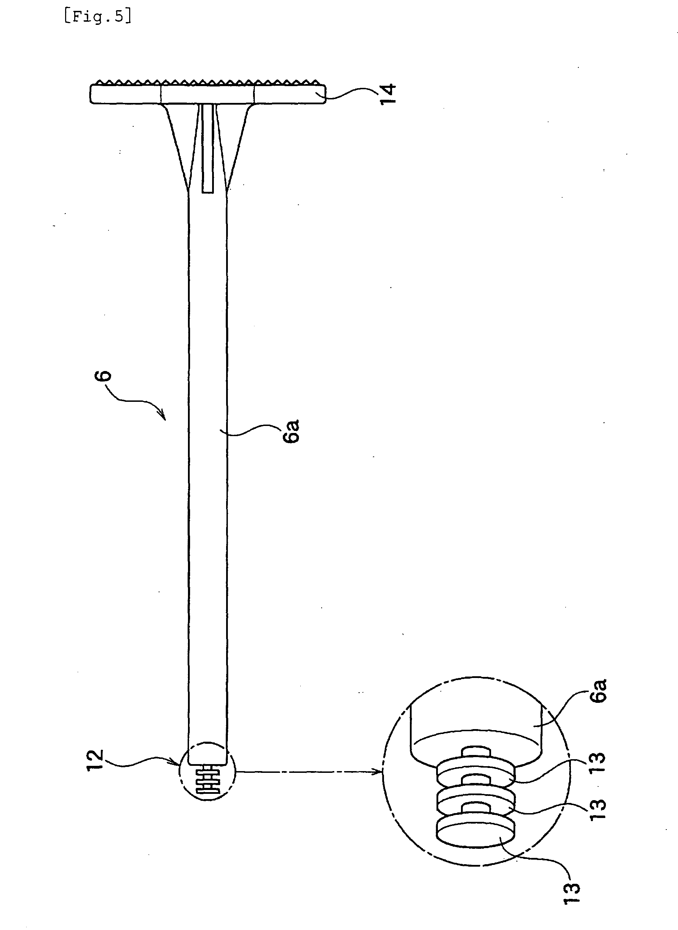

For the above embodiment for instance, the thread crest 12a in a spiral shape is formed on the male screw 12 of the plunger rod 6. However, it is unnecessary that the male screw 12 continues in a spiral shape, and a plurality of ring bodies can be simply disposed in a protruding manner at a predetermined interval as shown in Fig. 5.

In other words, a male screw in accordance with the present invention includes a protruding body other than a so-called thread crest that continues in a spiral shape. However, the screw shape as shown in Fig. 3 is preferable in particular from a point of view of a friction and damage to an inner wall of the depression of the piston.

Moreover, a plunger assembly for a syringe in which a piston having a small diameter is used is shown in the above embodiment. However, this does not prevent the present invention from being applied to a plunger assembly for a syringe in which a piston having a large diameter is used.

## Claims

1. A plunger assembly for a syringe, comprising:
a piston that is slidably inserted into a syringe barrel; and
a plunger rod in which one end part thereof is inserted into an opened depression formed on the base end side of the piston to support the piston in an integrated manner,
wherein the depression of the piston is a hole in a generally cylindrical shape formed and opened in one direction, a male screw that comes into contact with an inner wall of the cylindrical hole of the piston in a sliding manner or that is screwed into the hole is formed at one end part of the plunger rod, and the piston is supported to the plunger rod in an integrated manner by inserting the male screw part into the depression.

2. The plunger assembly for a syringe as defined in claim 1, wherein the piston is a laminate piston in which a resin film is laminated on the surface thereof.
